# FASCICULE DE BREVET EUROPEEN

(11) **EP 0 282 698 B1**
(45) Date de publication et mention de la délivrance du brevet: **14.10.1998**
(21) Numéro de dépôt: 88100839.5
(22) Date de dépôt: 21.01.1988
(51) Int. Cl.: A61K 9/52, A61K 31/54

(54) **Procédé pour la préparation d'un produit retard-contenant du diltiazem pour une administration journalière unique**
Verfahren zur Herstellung eines Retardpräparates, das Diltiazem in einer einzigen Tagesdosis enthält
Process for the preparation of a retarding formulation containing diltiazem for a single daily dose

(30) Priorité: 20.02.1987 CH 637/87
(43) Date de publication de la demande: 21.09.1988
(73) Titulaire: FARMARC NEDERLAND BV, 1007 JE Amsterdam (NL)
(72) Inventeur: Barth, Dieter, D-7904 Erbach (DE)
(74) Mandataire: Jörchel, Dietrich R.A.

(56) Documents cités:
- EP-A- 149 920
- WO-A-85/02114
- US-A- 4 173 626

## Description

Cette invention-ci concerne un produit retard contenant du diltiazem pour l'administration journalière unique. Le diltiazem (D-3-acétoxy-cis-2,3-dihydro-5-(2-diméthylaminoéthyl)-2-(p-méthoxy-phényl)-1,5-benzotiazepin-4(5H)-one) est un médicament appartenant au group des calcium-antagonistes, ayant la structure chimique suivante:

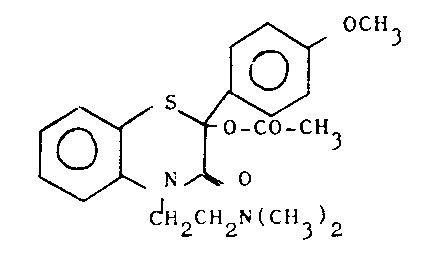

Sous la forme de chlorhydrate, il est amplement utilisé dans le traitement des cardiopathies coronariennes et de l'hypertension artérielle. La posologie moyenne des préparations orales est de 60 mg trois fois par jour. On trouve dans le commerce des préparations retard de 90 mg qui permettent l'administration d'une dose toutes les 12 heures (deux fois par jour); dans ces préparations la substance active est dégagée d'une façon relativement lente et constante, permettant d'obtenir des niveaux hématiques de médicament prolongés et efficaces. On a emploié les calcium-antagonistes (nifédipine, diltiazem, verapamil) avec beaucoup de succès, dès leur première introduction en thérapie, comme des médicaments anti-angineux actifs dans les différentes formes de maladie coronarienne (angine stable et instable, angine de Prinzmetal etc.).

Seulement par la suite on a mise en évidence la décisive efficacité de ces médicaments comme hypotenseurs, c'est-à-dire de substances actives dans les différentes formes d'hypertension artérielle. La nifédipine, en effect, a été le premier calcium-antagoniste qu'on a utilisé comme anti-hypertenseur et aujourd'hui elle est emploiée d'une maniére diffuse dans les thérapies avec cette indication.

L'autre calcium-antagoniste plus important, le diltiazem, introduit en thérapie plus tard que la nifédipine, a été lui-même, et il est au présent, largement emploié dans le traitement de l'angine de poitrine (Rosenthal S.J. et al.: "Efficacy of diltiazem for control of symptoms of coronary arterial spasm" - Am. J. Cardiol., 46: 1027-1032 (1980); Schroeden J.S. et al.: "Multiclinic controlled trial of diltiazem for Prinzmetal's angina" - Am. J. Med., 72; 227-232 (1982); Petru M.A. et al.: "Short and long-term efficacy of high-dose oral diltiazem for angina due to coronary artery disease" - Circulation, 68: 139-147 (1983); Hossack K, et al.: "Long-term study of highdose diltiazem in chronic stable exertional angina" - Am. Heart J., 207: 1215-1220 (1984)), mais depuis quelques années son emploi comme anti-hypertenseur, comme la nifédipine, est de plus en plus diffus (Aoki K. et al. "Hypotensive effects of diltiazem to normal and essential hypertensives" - Eur. J. Clin. Pharmacol., 25: 475-480 (1983); Safar M.E. et al. : "Hemodynamic effects of diltiazem in hypertension" - Circ. Res., 52 (suppl. I) : 169-173 (1983); Trimarco B. et al. : "Diltiazem in the treatment of mild to moderate essential hypertension" - J. Clin. Pharmacol., 24 :218-227 (1984); Lewis J.E. et al. : "Diltiazem in hypertension a preliminary dose-finding and efficacy multicenter study" - Curr. Ther. Res., 37 : 566-579 (1985) ; Yamauchi et al. : "Effects of diltiazem hydrochloride on cardiovascular response, plateiet aggregation and coagulating activity during exercise testing in systemic hypertension" - Am. J. Cardiol., 57 : 609-612 (1986)).

Le brevet EP 0 149 920 (ELAN CORPORATION), en partant de la méthode thérapeutique habituelle consistant à administrer le diltiazème par voie orale à des doses variant de 120 mg/jour en quatre doses de 30 mg, jusqu'à 240 mg/jour en trois à quatre doses, se propose de mettre au point une formulation-retard permettant de diminuer le nombre de prises quotidiennes à deux. L'invention selon EP 0 149 920, consiste en un pellet, composé d'un coeur constitué de diltiazème ou d'un de ses seis, d'un acide organique et d'un lubrifiant, et d'une membrane extérieure permettant une libération retardée du diltiazème. La vitesse de libération, mesurée en conditions standard ((pharmacopée) US XX, méthode de PADDLE)

| | |
|---|---|
| 0% - 10% du diltiazem | après deux heures |
| 10% - 30% | après quatre heures |
| 20% - 40% | après six heures |
| 50% - 80% | après huit heures |
| 85% - 100% | après douze heures. |

Dans le WO 85/02114 (ETHYPHARM) le problème technique posé est de créer une forme galénique orale de sulpiride permettant de réduire la prise journalière moyenne à deux gélules ou comprimés par jour, dosées en sulpiride à 100 mg par gélule ou comprimé, sinon à une gélule ou un comprimé par jour, dosée à 200 mg de sulpiride. Les meilleurs résultats, selon ce document, sont obtenus par un mode de réalisation (appelé forme III) dosé à 100 mg de sulpiride (2 gélules/jour). Cette forme est constituée d'un grain neutre, sur lequel est appliquée une couche de sulpiride/PVP, enrobé d'une couche extérieure de polymère microporeux, de préférence des métacrylates.

Dans le brevet US 4 173 626 (DEMPSKI) le problème technique posé est de créer une forme galénique orale d'indométhacine ayant un effet retard maximum, pour réduire au minimum le nombre de prises journalières, dans le traitement de troubles inflammatoires. L'invention selon Us 4 173 626 résoud ce problème à l'aide de pellets de 0,5 à 1,5 mm de diamètre, sans structure géométrique interne, constitué essentiellement d'indométhacine et d'un liant, en mélangeant dans une même capsule ces pellets, nus à ces même pellets enrobés par un agent à dissolution lente, l'acétate de polyvinyle. L'effet retard obtenu est d'une douzaine d'heures, ce qui correspond à deux prises quotidiennes.

Le but de cette invention est le développement d'une nouvelle préparation retard capable de permettre l'administration d'une dose unique toutes les 24 heures (une fois par jour), même si elle maintient des niveaux hématiques efficaces du médicament dans le cours de la journée entière.

La présente invention a pour objet un pellet de diltiazème tel que défini par la clause caractérisante de la revendication 1.

L'invention sera décrite au moyen de plusieurs exemples en se référant auf figures annexées dans lesquelles:
La figure 1.1 représente la courbe de dégagement d'un lot expérimental 15/S typique de la phase 1, à côté du produit de référence.
Les figures 1.2 et 1.3 représentent la courbe d'absorption "in vivo" d'une dose de 90 mg du même lot, respectivement d'une dose de 180 mg du produit expérimental comparée avec une dose de 2 comprimés de 90 mg du produit de référence.
La figure 2.1 représente la courbe de dégagement "in vitro" d'un lot expérimental 36/S typique de la phase 2 à côté du produit de référence.
La figure 2.2 représente les courbes des niveaux hématiques d'une dose unique de 90 mg et du produit de référence.
La figure 2.3 représente les relatives courbes d'absorption "in vivo".
La figure 3.1 représente la courbe de dégagement "in vitro" d'une préparation expérimentale typique (lot 11/c) de la phase 3, à côté de celle du produit de référence.
La figure 3.2 représente les courbes d'absorption du médicament dans l'homme.
La figure 4.1 représente les courbes de dégagement "in vitro" de quelques lots expérimentaux de la phase 4.
La figure 4.2 représente les courbes d'absorption de diltiazem dans l'homme.
La figure 4.3 représente les courbes hématiques du principal métabolite.

### PART EXPERIMENTALE

Chacun sait que le diltiazem est sujet à une rapide transformation métabolique ("first pass effect"), donc la biodisponibilité d'une dose orale est toujours inférieure au 100% et le pics hématiques ne sont pas linéairement proportionnels à la dose.

Avec une préparation à cession rapide, par exemple, une dose de 60 mg donne lieu à un pic hématique de 65 +/- 9 ng/ml, alors qu'une dose de 210 mg (3.5 fois) donne lieu à un pic hématique de 315 +/- 60 ng/ml (environ 5 fois) (Rovei V. et alia: Acta Cardiologica, 35: 35-45, 1980). C'est évident, donc, qu'avec des doses élevées les niveaux hématiques augmentent plus que proportionnellement, très probablement à cause d'une saturation métabolique qui neutralise en partie le "first pass effect". C'est pourquoi le développement de la préparation de cette invention-ci a été éxecuté en différentes phases, utilisant d'une fois à l'autre des différentes doses et des différentes courbes de dégagement et vérifiant par des études "in vivo", dans l'homme, les courbes hématiques d'absorption et la biodisponibilité des différentes préparations.

Toutes les préparations de cette invention-ci sont composées par des pellets contenus dans des capsules de gélatine dure. La préparation des pellets consiste à recouvrir, dans des cuvettes projetées à dessein, des petits granules d'excipient inerte ("neutral cores") par le principe actif opportunément mélangé avec des agents liants et dans la successive application d'une couche variable d'une membrane polymère de composition variée, à travers la quelle le principe actif même migre par diffusion lorsque les pellets se trouvent dans un milieu aqueux. En particulier, comme membranes de recouvrement on a utilisé les matériels suivants:
1) Shellac (gomme-laque)
2) Polymères à base d'acryl-métacrylates
3) Polymères cellulosiques comme éthylcellulose, hydroxiéthylcellulose, etc.

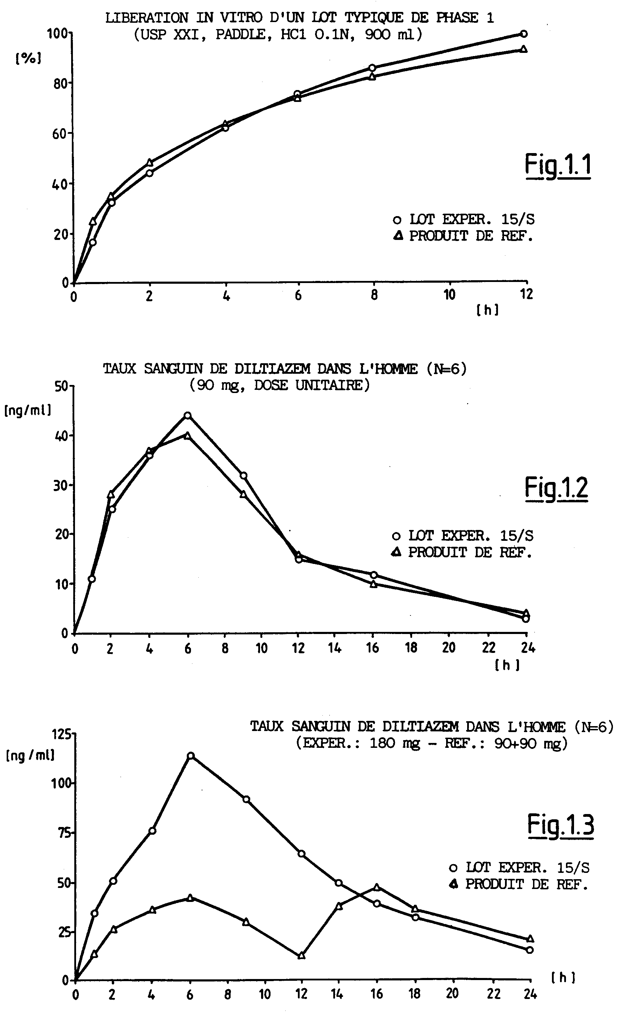

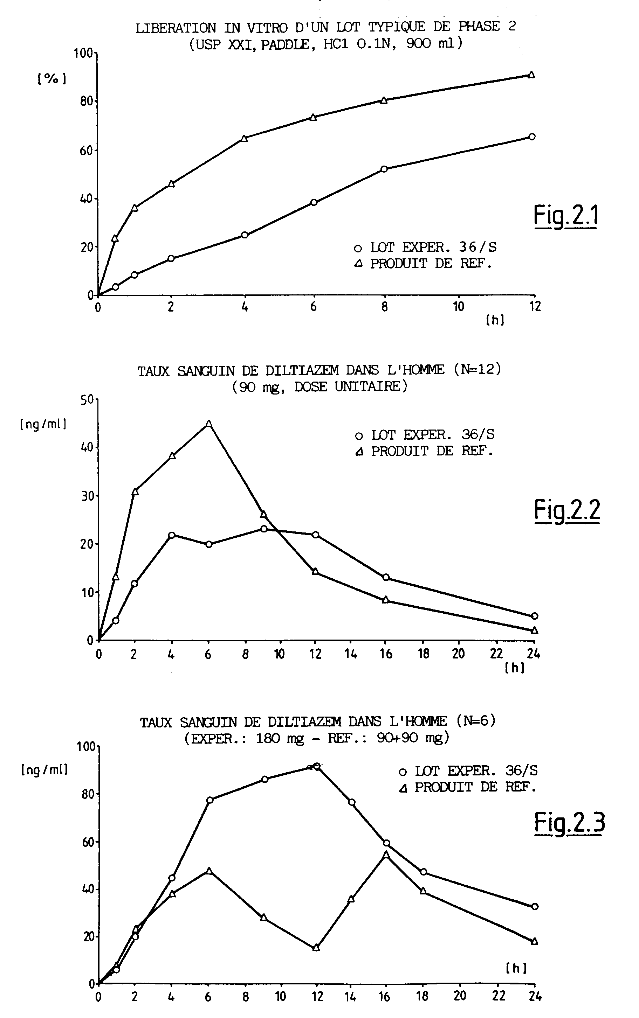

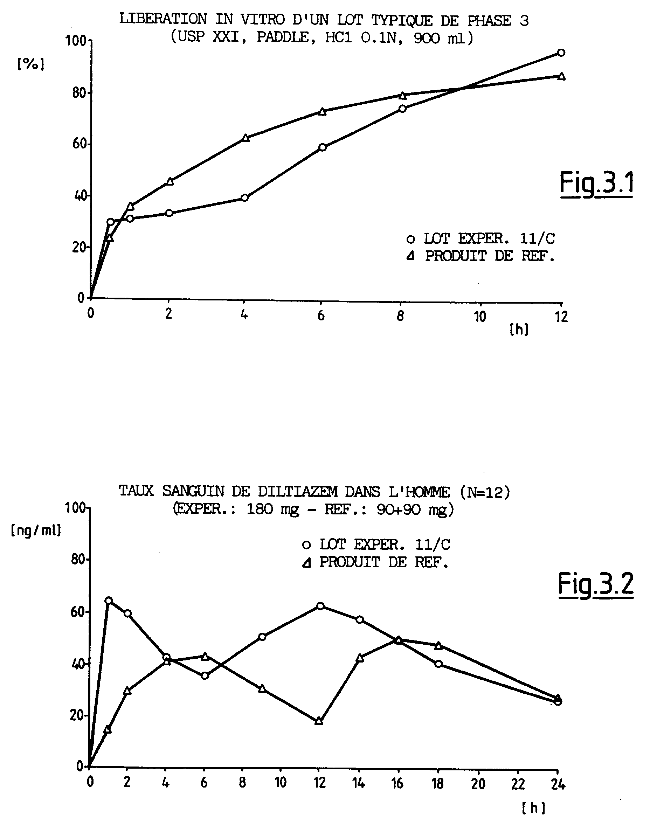

Dans tous les cas on a conditionné les pellets en capsules de gélatine dure de dimension 0, -1, -2 selon la dose.
PHASE 1: Dans cette phase on a procédé à la préparation de formulations retard de 90 et de 180 mg de diltiazem, avec dégagement "in vitro" le plus semblable possible à celui d'un produit retard analogue de 90 mg, qu'on a adopté pour tous les essais comme le produit de référence. Dans la figure 1.1. on a représenté la courbe de dégagement d'un lot expérimental 15/S typique de cette phase, à côté du produit de référence. Dans la figure 1.2 suivante on peut remarquer que la courbe d'absorption "in vivo" d'une dose de 90 mg du même lot est pratiquement superposable à celle du produit de référence. Dans la figure 1.3 on a représenté les courbes d'absorption "in vivo" d'une dose de 180 mg du produit expérimental comparée avec une dose de 2 comprimés de 90 mg (une comprimé toutes les 12 heures) du produit de référence.
   Dans ce cas, contrairement à ce qu'on à remarquè dans le cas précédent, les courbes hématique du produit expérimental dénotent une plus grande biodisponibilité du lot expérimental, avec des niveaux qui sont plus que proportionnels à la dose; néanmoins toutes les courbes hématiques sont caractérisées par une chute des niveaux à partir de la 4ème-6ème heure après l'administration, avec des niveaux pratiquement négligeables à la 24ème heure. Par cette raison, avec le but d'obtenir un plus grand retard avec des niveaux efficaces jusqu'à la 24ème heure, on est passé à la phase suivante.
PHASE 2: Dans cette phase on a procédé à la préparation de formulations retard de 90 mg et de 180 mg de diltiazem avec un dégagement "in vitro" nettement plus lent que celui du produit de référence et aussi plus lent que celui des lots expérimentals de la phase précédente. Dans la figure 2.1 on a représenté la courbe de dégagement "in vitro" d'un lot expérimental 36/S typique de cette phase, à côté du produit de référence. Dans la figure 2.2 on a représenté les courbes des niveaux hématiques, d'une dose unique de 90 mg dans l'homme, de la forme de 90 mg et du produit de référence, l'une et l'autre dans une dose unique. D'où l'on peut remarquer que le plus grand retard du lot expérimental se traduit pratiquement par une certaine perte de biodisponibilité, puisque la plus petite vitesse de dégagement donne lieu à une absorption plus lente et donc à une métabolisation plus efficace et relativement plus rapide. En d'autres termes, la vitesse de dégagement tend à s'approcher à la vitesse de "first pass effect", donc la courbe hématique se stabilise à un niveau assez bas.
   Nettement plus favorables, au contraire, sont les résultats obtenus par la dose de 180 mg de la même formulation expérimentale, comparée "in vivo" à une dose de 2 comprimés de 90 mg (une comprimé toutes les 12 heures) du produit de référence.
   Dans la figure 2.3 on a représenté les relatives courbes d'absorption "in vivo".
   Comme on peut remarquer par le développement des niveaux hématiques, la formulation expérimentale est nettement plus retardée que le produit de référence et les niveaux sont significatifs même à la 24ème heure; de plus les aires sous la courbe sont pratiquement équivalentes, ce qui dénote que la vitesse d'absorption d'une préparation lente comme celle de 90 mg, mais dans une dose plus haute c'est-à-dire 180 mg, dépasse nettement celle de métabolisation, donc la biodisponibilité n'est pas altérée. Néanmoins dans la courbe de la figure 2.3 le produit expérimental présente un développement excessivement lent dans la phase initiale, c'est pourquoi qu'il y a le doute que dans les premières 3-4 heures les niveaux hématiques de produit soient au-dessous des niveaux d'efficacité. Pour cette raison on a pensé qu'il fût opportun de préparer une nuovelle série de préparations expérimentales où une partie des pellets retardés a été replacée par des pellets à dégagement immédiat.
PHASE 3: Dans cette phase on a préparé des formulations retard "composites" de 180 mg, composées par une part variable du 60 au 95 % de pellets à cession retardée avec le dégagement typique de ceux de la phase 2 et pour la part restante du 40 au 5 % par des pellets à cession immédiate, c'est-à-dire pas recouverts par la mambrane retardante. Dans cette phase même, la comparaison "in vivo" a été faite avec le produit de référence administré avec la dose de deux comprimés de 90 mg (une comprimé toutes les 12 heures)!. Dans la figure 3.1 on a représenté la courbe de dégagement "in vitro" d'une préparation (lot 11/c) expérimentale typique (70 % pellets retardés + 30 % pellets rapides), à côté de celle du produit de référence, tandis que dans la figure 3.2 on a représenté les courbes d'absorption du médicament dans l'homme.. Les dernières démontrent clairement l'apparition précoce de niveaux efficaces de diltiazem, déjà 15-30 minutes après l'administration de la formulation expérimentale, provoqués par la rapide absorption du médicament des pellets à dégagement immediat, avec un pic à la 1ère heure; ensuite il y a une courte phase de diminution des niveaux, par la suite une rapide reprise et un nouveau pic à la 12ème heure et encore une graduelle diminution des niveaux jusqu'à la 24ème heure. A ce moment-là les niveaux sont encore suffisamment hauts et de peu plus petits que ceux du produit de référence administré en deux doses successives de 90 mg toutes les 12 heures. De plus de la même figure c'est évident que la biodisponibilité dans les 24 heures (aires sous la courbe) de la préparation expérimentale administrée en petite dose est équivalente à celle du produit de référence administré en deux doses, une toutes les 12 heures.
   Tout récemment, dans une étude sur la nifédipine, on a démontré que si le médicament arrive dans le sang d'une facon lente et graduelle, son efficacité anti-hypertenseuse est très marquée, tandis que si l'on arrive à des niveaux hématiques significatifs d'une facon rapide, l'effet anti-hypertenseur est très maigre. Ce démontre, en fait, que l'action anti-hypertenseuse d'un calcium-antagoniste, tel que la nifédipine, est de loin meilleure si la courbe hématique augmente d'une facon graduelle plutôt que rapide (Kleinbloesem C.H. et al.: "Rate of increase in the plasma concentrations of nifedipine as a major determinant of its hemodynamic effect in humans" - Clin. Pharmacol. Ther., 41: 26-30 (1987)).
   C'est donc évident, donnée la stricte analogie entre les deux médicaments (Frishman W.H. et al.: "Comparison of diltiazem and nifedipine for both angina pectoris and systemic hypertension" - Am. J. Cardiol., 56: 41H-46H (1985)), qu'aussi dans le cas du diltiazem on peut s'attendre à une meilleure efficacité anit-hxpertenseuse lorsque les niveaux hématiques présentent un accroissement lent et graduel.
   On rappele que dejà dans la Phase 2 on avait obtenu des résultats satisfaisants de biodisponibilité, quand bien même avec des courbes hématiques à traitement lent des syndromes angineuses.
   A la lumière des susdites nouvelles observations dans le domaine des maladies hypertenseuses, néanmoins, les préparations dejà décrites dans la susdite Phase 2 se révélent tout à fait aptes à l'emploi dans ce particulier domaine de la thérapie.
   Dans d'autres mots, les préparations de Phase 2, caractérisées par une très bonne biodisponibilité et par une montée lente et graduelle de la courbe hématique, se prêtent particulièrement bien au traitement des différentes formes d'hypertension artérielle.
   Pour vérifier et confirmer les données de la susdite Phase 2, on a entrepris une nouvelle serie d'essais propres à obtenir des préparations tou à fait semblables à celles décrites là-bas, c'est-à-dire avec des caractéristiques analogues tant "in vitro" que "in vivo" et l'on peut définir cette nouvelle phase de développement comme Phase Quatrième.
   Dans son cours on a réalisé soit des préparations d'échantillonnages expérimentales, soit des essais de biodisponibilité sur des volontaires adultes sains. Ci-après on décrit succinctement les résultats relatifs à ces essais.
Phase 4 - Dans cette phase on a préparé des nouvelles formulations, introduisant quelques variantes dans les procédés de préparation des pellets, avec le but de reproduir et perfectionner les caractéristiques précédemment obtenues avec les formulations réalisées dans la Phase 2.

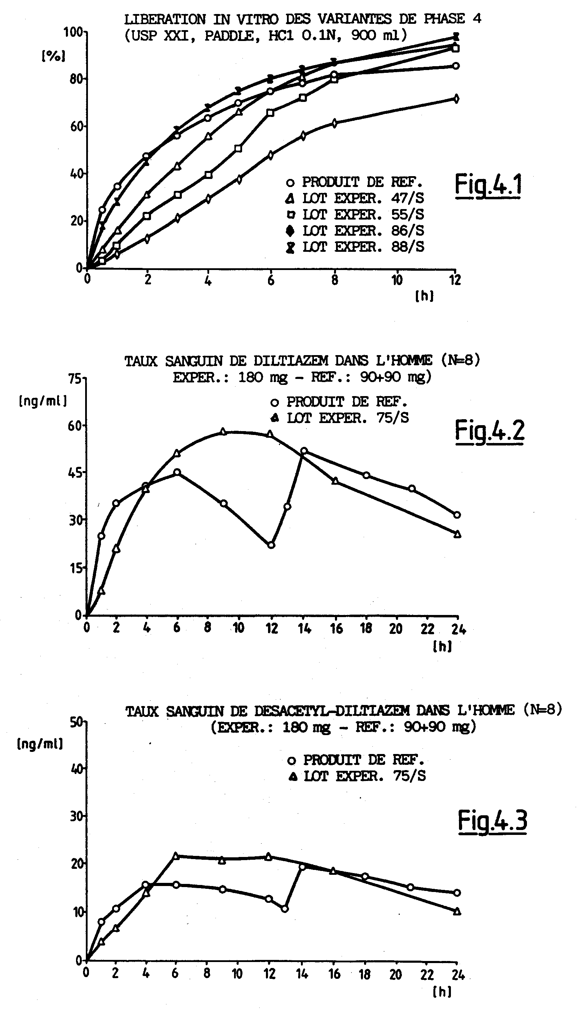

   Dans la figure 4.1 on a représenté les courbes de dégagement "in vitro" de quelques lots expérimentals préparés de cette façon, dans des différentes variantes technologiquement plus perfectionnées, par rapport aux courbes du même produit de référence dejà emploié précédemment dans la Phase 2. Avec le même produit de référence on a traité des volontaires sains adultes auxquels on a aussi administré un des nouveaux lots expérimentals, avec un traitement du type "cross-over". Dans la figure 4.2 on a représenté les courbes d'absorption de diltiazem dans l'homme après tels traitements.
   Dans la figure 4.3 on a représenté les courbes hématiques du principal métabolite, le desacétyl-diltiazem, dans les mêmes sujets de l'essai susdit. La courbe reproduite dans la figure 4.2 prouve que les niveaux hématiques de diltiazem, suivant une dose unique de médicament administré avec la formulation en pellets, sont très semblables à ceux obtenus avec deux doses journalières (une toutes les 12 heures) du produit de référence.
   Un développement pratiquement identique est présenté par les niveaux du métabolite desacétyl-diltiazem (fig. 4.3) quand bien même avec des valeurs absolues nettement plus basses.
   Les courbes de la Phase 4 qu'on vient de citer preuvent d'être tout à fait analogues à celles de la Phase 2 et donc propres à conférer au produit diltiazem ainsi formulé une efficacité décisive dans le traitement de l'hypertension artérielle.

### EXEMPLES

Les exemples explicatifs suivants indiquent de facon pas limitative la préparation des pellets de diltiazem dans les deux formes à dégagement rapide et à dégagement retardé.

### EXEMPLE A

### A.1 PELLETS RAPIDES DE DILTIAZEM

4.00 Kg de diltiazem et 1.00 Kg de talc, avec une granulométrie de 40 à 80 micron, sont mélangés sur un tourne-fûts.

1.50 Kg de microgranules composés par saccharose et amidon, avec une granulométrie de 0.500.0.710 mm, sont mis à tourner dans une cuvette avec un panier de 450 mm. Sur la masse tournante on pulvérise, par une speciale pompe à membrane, 30 g d'une solution au 40 % de gomme-laque (*) en alcool éthylique et on asperge avec 100 g du susdit mélange.
(*) (Gomme-laque type: poudre trois cercles extra decirée-blanchie).

L'operation du pulvérisation et d'aspersion est alternée et est répétée 50 fois en total.

### A.2 PELLETS RETARDÉS DE DILTIAZEM

La quantité de pellets obtenue (point A.1) est mise à tourner dans une cuvette de 450 mm de diamètre et baignée avec une solution de gomme-laque pulvérisée en éthanol (40 %) 40 g et aspergée avec 90 g de talc.

L'operation de pulverisation et d'aspersion est alternée et est répétée 25 fois en total.

### EXEMPLE B

### B.1 PELLETS RAPIDES DE DILTIAZEM

2.00 Kg de microgranules composés par saccharose et amidon avec une granulométrie de 0.500-0.710 mm sont mis à tourner dans une cuvette en acier inoxydable du diamètre de 450 mm. Sur la masse tournante on pulvérise, par une speciale pompe doseuse à membrane, 42 g d'une solution au 12.5 % de polymétacrylates (Eudragit L marque déposée) en acétone-éthanol et on asperge avec 40 g de diltiazem avec une granulométrie de 40-80 micron. L'operation de pulverisation et d'aspersion est alternée et est répétée 100 fois en total.

### B.2 PELLETS PETARDÉS DE DILTIAZEM

La quantité de pellets obtenue (point B.1) est mise à tourner dans une cuvette de 450 mm de diamètre et baignée avec une solution pulvérisée au 14.5 % de polymétacrylates (Eudragit S marque déposée) en acétone-éthanol et aspergée avec 65 g de talc.

L'operation de pulvérisation et d'aspersion est alternée et est répétée 20 fois en total.

### EXEMPLE C

### C.1 PELLETS RAPIDES DE DILTIAZEM

2.00 Kg de microgranules composés par saccharose et amidon avec une granulométrie de 0.500-0.710 mm sont mis à tourner dans une cuvette avec un panier en acier inoxydable de 450 mm de diamètre. Sur la messe tournante on pulvérise, par une pompe doseuse à membrane, 26 g d' une solution au 40 % de gomme-laque en éthanol et on asperge avec 80 g de diltiazem avec une granulométrie de 40-80 micron.

L'operation de pulvérisation et d'aspersion est alternée et est répétée 50 fois en total.

### C.2 PELLETS RETARDÉS DE DILTIAZEM

La quantité de pellets obtenue (point C.1) est mise à tourner dans une cuvette d'un diamètre de 450 mm et baignée avec 80 g d'une solution pulvérisée au 5 % d' ethoxyl N 100 (marque déposée) en acétone-éthanol et aspergée avec 54 g de talc. L'operation de pulvérisation et d'aspersion est alternée et est répétée 25 fois en total.

### EXEMPLE D

### PELLETS DE DILTIAZEM VARIANTE 47/S

4.00 Kg de diltiazem et 1.00 Kg de talc, avec une granulométrie de 40 à 80 micron, sont mélangés sur une tourne-fûts.

1.50 Kg de microgranules neutres composés par saccharose et amidon avec une granulomètrie de 0.500 à 0.710 mm sont mis à tourner dans une cuvette avec un panier de 450 mm.

Sur la masse tournante on pulvérise par une speciale pompe à membrane 25 g d'une solution de Kollidon 30 (marque déposée) au 20 % d'alcool éthylique et on asperge avec 50 g du susdit mélange.

L'operation de pulvérisation et d'aspersion est alternée et est répétée 100 fois en total. À ce point on pulvérise sur la masse tournante 80 g d'une solution au 5 % d'Ethoxyl M 100 (marque déposée) en acétone-éthanol et on asperge avec 50 g de talc. L'operation de pulvérisation et d' aspersion est alternée et est répétée 20 fois en total.

### EXEMPLE E

### PELLETS DE DILTIAZEM VARIANTE 85/S

4.00 Kg de diltiazem et 1.00 Kg de talc, avec une granulomètrie de 40 à 80 micron, sont mélangés sur un tourne-fûts.

1.50 Kg de microgranules neutres composés par saccharose et amidon avec une granulomètrie de 0.500 à 0.710 mm sont mis à tourner dans une cuvette avec un panier de 450 mm.

Sur la masse tournante on pulvérise par une speciale pompe à membrane 25 g d'une solution de Carbowax 4000 (marque déposée) au 20 % d'alcool éthylique et on asperge avec 50 g du susdit mélange.

L'operation de pulvérisation et d'aspersion est alternée et est répétée 100 fois en total. À ce point on pulvérise sur la masse tournante 100 g d'une solution au 5 % d'Ethoxyl T 100 (marque déposée) en acétone-éthanol et on asperge avec 50 g de talc. L'operation de pulvérisation et d' aspersion est alternée et est répétée 20 fois en total.

## Revendications

1. Pellet de diltiazème à libération retardée comprenant un pellet à libération rapide, ledit pellet à libération rapide étant constitué de diltiazème ou d'un chlorhydrate de diltiazème en association avec au moins un liant, et une membrane extérieure à travers laquelle le dégagement du diltiazème peut s'effectuer dans un milieu aqueux, caractérisé en ce que la courbe de dégagement "in vitro" du diltiazème, contrôlée selon la méthode de PADDLE de la Pharmacopée US XXI présente un profil approximativement linéaire (36/S, 86/S, 55/S) pendant les 6 premières heures, dont la pente est comprise entre 6% par heure et 11% par heure, et que ledit dégagement s'effectue de façon lente et graduelle, de sorte que le taux de dégagement total après 12 heures soit compris entre 65% et 93% de la quantité totale de diltiazème contenu dans ledit pellet.

2. Pellet de diltiazème à libération retardée selon la revendication 1, caractérisé en ce que ladite courbe de libération (36/S) présente les caractéristiques numériques approximatives :
| | | |
|---|---|---|
| a) Libération de | 15% du diltiazème total | après 2h. |
| b) | 25% | après 4h. |
| c) | 38% | après 6h. |
| d) | 52% | après 8h. |
| e) | 65% | après 12h. |

3. Pellet de diltiazème à libération retardée selon l'une des revendications 1 ou 2, caractérisé en ce que ledit pellet rapide comprend un granule central inerte entouré d'une pluralité de couches de diltiazème et de liant (s) et que ladite membrane est constituée d'une pluralité de couches de talc et de polymères.

4. Pellet de diltiazème à libération retardée selon la revendication 3, caractérisé en ce que ledit pellet rapide susceptible d'être obtenu par un procédé comprenant les opérations suivantes :
a) Faire tourner lesdits granules dans un appareil d'enrobage ;
b) Pulvériser au moins un liant en solution dans un solvant organique choisi parmi l'éthanol et les mélanges éthanol-acétone sur lesdits granules ;
c) Poudrer les granules ayant subi la pulvérisation avec une poudre comprenant essentiellement du diltiazème ou un chlorhydrate de diltiazème.
d) Répéter les opérations b) et c) alternativement de l'ordre de 50 à 100 fois.

5. Pellet de diltiazème à libération retardée selon la revendication 4, caractérisé en ce que le matériau dudit granule est un mélange d'amidon et de saccharose, que ledit solvant organique est choisi parmi l'éthanol et les mélanges acétone-éthanol et que ledit liant est choisi parmi la gomme-laque et les polymétacrylates.

6. Pellet de diltiazème à libération retardée selon l'une des revendications 4 ou 5, caractérisé en ce que ladite membrane est susceptible d'être réalisée à l'aide d'un procédé comprenant les opérations suivantes :
e) Pulvériser sur les pellets rapides une solution d'un polymère dans un solvant organique choisi parmi l'éthanol et les mélanges éthanol-acétone ;
f) Poudrer les pellets rapides ayant subi la pulvérisation à l'aide de talc ;
g) Répéter les opérations e) et f) alternativement de l'ordre de 20 à 25 fois.

7. Pellet de diltiazème à libération retardée selon la revendication 6, caractérisé en ce que ledit solvant organique est choisi parmi l'éthanol et des mélanges éthanol-acétone, que lesdits polymères constituant ladite membrane sont choisis parmi la gomme-laque, les acrylmétacrylates et les polymères cellulosiques.

8. Utilisation de pellet de diltiazème à libération retardée selon l'une quelconque des revendications précédentes pour la fabrication d'une forme orale solide destinée au traitement de l'hypertension artérielle ou de l'angine de poitrine par administration de doses uniques quotidiennes

9. Utilisation de pellet de diltiazème à libération retardée selon la revendication 8, pour la fabrication d'une forme orale solide en capsules de gélatine dure destinées à l'administration de doses uniques quotidiennes, pour le traitement de l'hypertension.

10. Utilisation selon la revendication 9, caractérisée en ce que ladite capsule contient l'équivalent de 120 à 360 mg de diltiazème.

11. Utilisation selon la revendication 9, caractérisée en ce que ladite capsule contient l'équivalent de 180mg de diltiazème.

12. Utilisation d'un mélange d'un pellet de diltiazème à libération retardée selon l'une des revendications 1 à 7 et de pellets de diltiazème à libération rapide pour la fabrication d'une forme orale solide destinée au traitement de troubles coronariens, et notamment l'angine de poitrine, par administration de doses uniques quotidiennes.

13. Utilisation d'un mélange de pellets de diltiazème à libération retardée et de pellets de diltiazème à libération rapide selon la revendication 12 comprenant de 60% à 95% de pellets à libération retardée et de 40 à 5% de pellets à libération rapide.

14. Utilisation d'un mélange de pellets selon la revendication 13 pour la fabrication d'une forme orale solide en capsules, ladite capsule contenant l'équivalent de 120 à 360 mg de diltiazème.

15. Utilisation selon la revendication 14, caractérisée en ce que ladite capsule contient l'équivalent de 180 mg de diltiazème

## Claims

1. Diltiazem pellet with retarded release including a rapid release pellet, the said rapid release pellet consisting of diltiazem or a hydrochloride of diltiazem in association with at least one binder, and an outer membrane through which release of the diltiazem can take place in an aqueous medium, characterized in that the "in vitro" release curve of the diltiazem, determined by the PADDLE method of the US Pharmacopoeia XXI has an approximately linear profile (36/S, 86/S, 55/S) during the first 6 hours, the slope of which lies between 6% per hour and 11% per hour, and in that the said release occurs slowly and gradually, so that the total rate of release after 12 hours lies between 65% and 93% of the total quantity of diltiazem contained in the said pellet.

2. Diltiazem pellet with retarded release according to claim 1, characterized in that the said release curve (36/S) has the approximate numerical characteristics :
| | | |
|---|---|---|
| a) Release of | 15% total diltiazem | after 2 h. |
| b) | 25% | after 4 h. |
| c) | 38% | after 6 h. |
| d) | 52% | after 8 h. |
| e) | 65% | after 12 h. |

3. Diltiazem pellet with retarded release according to either of claims 1 or 2, characterized in that the said rapid pellet comprises an inert central granule surrounded by a plurality of layers of diltiazem and binder(s) and in that the said membrane consists of a plurality of layers of talc and polymers.

4. Diltiazem pellet with retarded release according to claim 3, characterized in that the said rapid pellet can be obtained by a process comprising the following operations :
a) Causing the said granules to revolve in a coating apparatus;
b) Spraying at least one binder in solution in an organic solvent chosen from ethanol and ethanol-acetone mixtures onto the said granules;
c) Powdering the granules that have undergone spraying, with a powder comprising essentially diltiazem or a hydrochloride of diltiazem;
d) Repeating the operations b) and c) alternately approximately 50 to 100 times.

5. Diltiazem pellet with retarded release according to claim 4, characterized in that the material of the said granule is a mixture of starch and sucrose, that the said organic solvent is chosen from ethanol and acetone-ethanol mixtures, and that the said binder is chosen from shellac and polymethacrylates.

6. Diltiazem pellet with retarded release according to either of claims 4 or 5, characterized in that the said membrane can be produced by means of a process comprising the following operations :
e) Spraying the rapid pellets with a solution of a polymer in an organic solvent chosen from ethanol and ethanol-acetone mixtures ;
f) Powdering with talc the rapid pellets that have undergone spraying ;
g) Repeating the operations e) and f) alternately approximately 20 to 25 times.

7. Diltiazem pellet with retarded release according to claim 6, characterized in that the said organic solvent is chosen from ethanol and ethanol-acetone mixtures and that the said polymers constituting the said membrane are chosen from shellac, acrylmethacrylates and cellulosic polymers.

8. Use of a diltiazem pellet with retarded release according to any of the preceding claims for the manufacture of a solid oral form intended for the treatment of arterial hypertension or angina pectoris by the administration of single daily doses.

9. Use of a diltiazem pellet with retarded release according to claim 8, for the manufacture of a solid oral form made of hard gelatine capsules intended for the administration of single daily doses for the treatment of hypertension.

10. Use according to claim 9, characterized in that the said capsule contains the equivalent of 120 to 360 mg of diltiazem.

11. Use according to claim 9, characterized in that the said capsule contains the equivalent of 180 mg of diltiazem.

12. Use of a mixture of a diltiazem pellet with retarded release according to one of claims 1 to 7 and diltiazem pellets with rapid release for the manufacture of a solid oral form intended for the treatment of coronary disorders, and in particular angina pectoris, by the administration of single daily doses.

13. Use of a mixture of diltiazem pellets with retarded release and diltiazem pellets with rapid release according to claim 12 comprising 60% to 95% of pellets with retarded release and 40% to 5% of pellets with rapid release.

14. Use of a mixture of pellets according to claim 13 for the manufacture of a solid oral form in capsules, the said capsule containing the equivalent of 120 to 360 mg of diltiazem.

15. Use according to claim 14, characterized in that the said capsule contains the equivalent of 180 mg of diltiazem.

## Patentansprüche

1. Diltaizempellet zur verzögerten Freisetzung mit einer Pellet zur schnellen Freisetzung, die aus Diltiazem oder einem Diltiazem-Chlorhydrat in Verbindung mit eine Bindemittel besteht, mit einer äußeren Membran, durch die die Abgabe des Diltiazems in eine wässrigen Milieu stattfinden kann, dadurch gekennzeichnet, daß die Abgabekurve des Diltiazems "in vitro" gesteuert nach dem PADDLE-Verfahren der US Pharmakopöe XXI während der ersten sechs Stunden im wesentlichen ein lineares Profil (36/S, 86/S, 55/S) aufweist, dessen Abfall zwischen 6 % und 11 % pro Stunde liegt, und daß die Abgabe langsam und allmählich so erfolgt, daß die gesamte Abgabemenge nach 12 Stunden zwischen 65 % und 93 % der in dem Pellet enthaltenen Gesamtmenge an Diltiazem liegt.

2. Diltiazempellet zur verzögerten Freisetzung nach Anspruch 1, dadurch gekennzeichnet, daß die Abgabekurve (36/S) die angenäherten zahlenmäßigen Charakteristiken aufweist:
| | | |
|---|---|---|
| a) Freisetzung von | 15 % des Gesamt-Diltiazems | nach 2 Stunden |
| b) | 25 % | nach 4 Stunden |
| c) | 38 % | nach 6 Stunden |
| d) | 52 % | nach 8 Stunden |
| e) | 65 % | nach 12 Stunden |

3. Diltiazempellet zur verzögerten Freisetzung nach Anspruch 1 oder 2, dadurch gekennzeichnet, daß das schnelle Pellet ein inertes zentrales Teil aufweist, das mit einer Mehrzahl von Diltiazem- und Bindemittelschichten umgeben ist und daß die genannte Membran aus einer Mehrzahl von Talg- und Polymerschichten besteht.

4. Diltiazempellet zur verzögerten Freisetzung nach Anspruch 3, dadurch gekennzeichnet, daß das schnelle Pellet durch ein Verfahren mit folgenden Verfahrensschritten erhältlich ist:
a) Rotieren dar genannten Teilchen in einem Beschichtungsapparat,
b) Aufspritzen wenigstens eines Bindemittels in Lösung in einem organischen Lösungsmittel, das aus Ethanol und Ethanol-Aceton-Mischungen ausgewählt ist, auf die genannten Teilchen,
c) Einstäuben der Teilchen nach dem Aufspritzen mit einem Puder, das im wesentlichen aus Diltiazem oder einem Diltiazem-Chlorhydrat besteht,
d) abwechselndes Wiederholen der Verfahrensschritte b) und c) größenordnungsmäßig 50 bis 100 mal.

5. Diltiazempellet zur verzögerten Freisetzung nach Anspruch 4, dadurch gekennzeichnet, daß das Material der Teilchen eine Mischung aus Stärke und Saccharose ist, daß das organische Lösungsmittel aus Ethanol oder Aceton-Ethanol-Mischungen ausgewählt ist und daß das Bindemittel aus Gummilack und den Polymetacrylaten ausgewählt ist.

6. Diltiazempellet zur verzögerten Freigabe nach Anspruch 4 oder 5 dadurch gekennzeichnet, daß die genannte Membran durch ein Verfahren mit folgenden Verfahrensschritten herstellbar ist:
e) Aufspritzen einer Lösung eines Polymers in einem organischen Lösungsmittel, das aus Ethanol und Ethanol-Aceton-Mischungen ausgewählt ist, auf die schnellen Pellets,
f) Bepudern der schnellen Pellets nach dem Aufspritzvorgang mit Talg,
g) abwechselndes Wiederholen der Verfahrensschritte e) und f) etwa 20 bis 25 mal.

7. Diltiazempellets zur verzögerten Freigabe nach Anspruch 6, dadurch gekennzeichnet, daß das organische Lösungsmittel aus Ethanol und Ethanol-Aceton-Mischungen ausgewählt ist, daß die genannten die Membran bildenden Polymere aus Gummilack, Acryl mit Acrylaten und Zellulosepolymeren ausgewählt sind.

8. Verwendung eines Diltiazempellets zur verzögerten Freisetzung nach einem der vorhergehenden Ansprüche zur Herstellung einer festen oralen Form zur Behandlung der arteriellen Hypertonie oder der Angina pectoris durch Verabreichung einer einzigen Dosis täglich.

9. Verwendung eines Diltiazempellets zur verzögerten Freisetzung nach Anspruch 8 zur Herstellung einer festen oralen Form als harte Gelantinekapsel zur Verabreichung einer einzigen täglichen Dosis zur Behandlung der Hypertonie.

10. Verwendung nach Anspruch 9, dadurch gekennzeichnet, daß die Kapsel ein Äquivalent von 120 bis 360 mg Diltiazem enthält.

11. Verwendung nach Anspruch 9, dadurch gekennzeichnet, daß die Kapsel ein Äquivalent von 180 mg Diltiazem enthält.

12. Verwendung einer Mischung einer Diltiazempellet zur verzögerten Freisetzung nach eine der Ansprüche 1 bis 7 und von Diltiazemtpellet zur schnellen Freisetzung zur Herstellung einer festen oralen Form zur Behandlung von Koronarbeschwerden, insbesondere der Angina pectoris, durch Verabreichung einer einzigen täglichen Dosis.

13. Verwendung einer Mischung von Diltiazempellets zur verzögerten Freisetzung und Diltiazempellets zur schnellen Freisetzung nach Anspruch 12 enthaltend 60 bis 95 % an Pellets zur verzögerten Freisetzung und 40 bis 5 % von Pellets zur schnellen Freisetzung.

14. Verwendung einer Mischung von Pellets nach Anspruch 13 zur Herstellung einer festen oralen Form in Kapseln, die ein Äquivalent von 120 bis 360 mg Diltaizem enthalten.

15. Verwendung nach Anspruch 14, dadurch gekennzeichnet, daß die Kapsel das Äquivalent von 180 mg Diltiazem enthalten.
